# EUROPEAN PATENT APPLICATION

(11) **EP 4 703 004 A1**
(43) Date of publication of application: **04.03.2026**
(21) Application number: 24796284.8
(22) Date of filing: 26.04.2024
(51) Int. Cl.: A61N 1/362

(54) **MEDICAL INSTRUMENT**

(30) Priority: 26.04.2023 CN 202310465860
(71) Applicant: United Innomed (Shanghai) Limited, Shanghai 201315 (CN)
(72) Inventor: WANG, Li, Shanghai 201315 (CN); SUN, Qingyi, Shanghai 201315 (CN)
(74) Representative: Barker Brettell LLP
(86) International application number: PCT/CN2024/090200
(87) International publication number: WO 2024/222915

(57) **Abstract**

A medical instrument, comprising a control apparatus. The control apparatus is configured to execute the following steps: within an operation period, acquiring an in-vivo near-field cardiac electrical by an electrode pair (S001); acquiring an cardiac electrical sensing time (S002); and according to the cardiac electrical sensing time, estimating a start time or sensing time of a far-field R-wave within the operation period (S003). The medical instrument can estimate a start time or sensing time of a far-field R-wave within an operation period, such that a cardiac treatment can be provided for a patient at the right time, thereby improving the treatment effect and safety of the patient. Since it is no longer essential to depend on a real-time in-vivo far-field myocardial cardiac electrical or in-vitro far-field cardiac electrical, the structure of the medical instrument can be simplified, thereby reducing the energy consumption.

## Description

The present application claims priority to Chinese Patent Application CN202310465860.1, filed on April 26, 2023. The contents of the Chinese patent application are incorporated herein by reference in its entireties.

### TECHNICAL FIELD

The present application relates to the technical field of medical instruments, and in particular to a medical instrument having a control device.

### BACKGROUND

In medical apparatuses that provides cardiac therapy, the time, duration (P/R-waves and absolute refractory periods) and sequence of atrial and ventricular electrical activation are often critical to the operation, effectiveness and safety of the medical apparatuses. In an example of a cardiac pacemaker, the timing of atrial and/or ventricular electrical activities forms the basis for their pacing operations. In an example of a cardiac contractility modulation (CCM) therapy device, CCM pulses are typically delivered about 30-40 ms after a sensed near-field R-wave (local sense, LS), to ensure that the time when the pulse passes to the myocardium (where a R-sensed event has occurred) is within and early in an absolute refractory period for local myocardial depolarization. On the other hand, for current cardiac apparatuses (such as pacemakers, CCM apparatuses, etc.), electrode leads typically only sense local atrial and/or ventricular electrical activation. However, the device generally does not know the start time and duration of the entire atrial and/or ventricular activation, and a time window for safe delivery of CCM pulses during the electrical activities originating from the atrium and ventricle. Once the above information is obtained, the CCM pulses can be delivered to one or more stimulation sites in a safe and effective manner within the same heartbeat. Specifically, a time window for safe delivery of CCM pulses can be determined based on the overall ventricular electrical activity (R-wave) in an in-vivo far-field myocardial electrogram or an in-vitro far-field cardiac electrogram, and can cover an absolute refractory period of the entire ventricular myocardium. In addition, considering the duration of the overall ventricular electrical activity (R-wave), additional confidence can also be provided, that is, a plurality of LSs are confirmed as part of the overall ventricular activation of the same beat, reducing the likelihood that other non-R-wave related activities, such as T-waves, muscle noise, or other signals, cause oversensing.

However, after implantation of an associated medical instrument, an in-vivo far-field myocardial electrogram or an in-vitro far-field cardiac electrogram and an in-vivo near-field myocardial electrogram are obtained, which increases the complexity of hardware and software of the apparatus and the loss of the capabilities of associated components, and in the event of a malfunction of the apparatus, the medical instrument may not operate properly. On the other hand, adding a plurality of additional electrodes to obtain a far-field myocardial electrogram or an in-vitro far-field cardiac electrogram for a patient requiring the use of a pacemaker or another medical instrument can also result in an increased burden on the patient, and may cause many problems in practical applications.

### CONTENT OF THE PRESENT INVENTION

A first aspect of the present application provides a medical instrument, including a control device configured to perform the following steps:
acquiring an in-vivo near-field cardiac electrogram by an electrode pair within an operational period;
acquiring a cardiac electrical sensing time; where the cardiac electrical sensing time is a sensing time corresponding to a sensed event in the in-vivo near-field cardiac electrogram; and
estimating a start time (Br_OP) or a sensing time (GS_OP) of a far-field R-wave within the operational period based on the cardiac electrical sensing time.

Optionally, the electrode pair is a first electrode pair, the cardiac electrical sensing time is a first sensing time (vLS_OP), the in-vivo near-field cardiac electrogram is a first in-vivo near-field cardiac electrogram, and the sensed event is a near-field R-wave.

Optionally, the control device is further configured to perform the following step: estimating the start time (Br_OP) or the sensing time (GS_OP) of the far-field R-wave within the operational period based on the first sensing time (vLS_OP), a second sensing time (vLS_SUP) and a third sensing time (GS_SUP); where the second sensing time (vLS_SUP) is a sensing time corresponding to a sensed event in a first in-vivo near-field cardiac electrogram acquired by the first electrode pair within a set up period, and the third sensing time (GS_SUP) is a sensing time of an R-wave in a far-field cardiac electrogram acquired within a set up period.

Optionally, the control device is further configured to perform the following steps:
within the set up period or the operational period, obtaining a second constant (C2) based on the second sensing time (vLS_SUP), the third sensing time (GS_SUP) and a first time difference value (C1) between the third sensing time (GS_SUP) and a start time (Br_SUP) of the far-field R-wave within the set up period; and
within the operational period, estimating the start time (Br_OP) of the R-wave in the far-field cardiac electrogram within the operational period based on the first sensing time (vLS_OP) and the second constant (C2).

Optionally, the control device is further configured to perform the following steps:
within the set up period or the operational period, obtaining a second constant (C2) based on the second sensing time (vLS_SUP), the third sensing time (GS_SUP) and a first constant (C1); and
within the operational period, estimating the start time (Br_OP) of the far-field R-wave within the operational period based on the first sensing time (vLS_OP) and the second constant (C2).

Optionally, the control device is further configured to perform the following steps:
obtaining a third time difference value (C3) between a second sensing time (vLS_SUP) and a third sensing time (GS_SUP) within a set up period or the operational period, wherein the second sensing time (vLS_SUP) is a sensing time corresponding to a sensed event in the first in-vivo near-field cardiac electrogram acquired by the first electrode pair within the set up period, and the third sensing time (GS_SUP) is a sensing time of an R-wave in a far-field cardiac electrogram acquired within a set up period; and
estimating the sensing time (GS_OP) of the far-field R-wave within the operational period based on the first sensing time (vLS_OP) and the third time difference value (C3) within the operational period.

Optionally, the control device is further configured to perform, during the operational period, the following step: within the operational period, estimating the start time (Br_OP) of the R-wave in the far-field cardiac electrogram within the operational period based on the estimated sensing time (GS_OP) of the R-wave in the far-field cardiac electrogram within the operational period and a first constant (C1), wherein the first constant is in the range of 0-100 ms.

Optionally, the control device is further configured to perform the following step: estimating the start time (Br_OP) or the sensing time (GS_OP) of the far-field R-wave within the operational period based on the first sensing time (vLS_OP), a second sensing time (vLS_SUP) and a start time (Br_SUP) of the far-field R-wave within a set up period; where the second sensing time is a sensing time corresponding to a sensed event in the first in-vivo near-field cardiac electrogram acquired by the first electrode pair within a set up period.

Optionally, the control device is further configured to perform the following steps:
within a set up period or the operational period, obtaining a fourth time difference value (C4) between a second sensing time (vLS_SUP) and a start time (Br_SUP) of the far-field R-wave within the set up period; where the second sensing time (vLS_SUP) is a sensing time corresponding to a sensed event in the first in-vivo near-field cardiac electrogram acquired by the first electrode pair within the set up period; and
within the operational period, estimating the start time (Br_OP) of the far-field R-wave within the operational period based on the first sensing time (vLS_OP) and the fourth time difference value (C4).

Optionally, the electrode pair is a second electrode pair, the cardiac electrical sensing time is a fourth sensing time (aLS_OP), the in-vivo near-field cardiac electrogram is a second in-vivo near-field cardiac electrogram, and the sensed event is a near-field P-wave; and
where the control device is further configured to perform the following steps:
acquiring a first sensing time (vLS_OP); where the first sensing time (vLS_OP) is a sensing time corresponding to a sensed event in a first in-vivo near-field cardiac electrogram acquired by a first electrode pair within an operational period;
determining a time interval (PR_OP) between a near-field P-wave and a near-field R-wave within the operational period based on the first sensing time (vLS_OP) and the fourth sensing time (aLS_OP); and
estimating the start time (Br_OP) or the sensing time (GS_OP) of the far-field R-wave within the operational period based on the fourth sensing time (aLS_OP) and the time interval (PR_OP) between the near-field P-wave and the near-field R-wave within the operational period.

Optionally, the control device is further configured to perform the following steps:
within a set up period or the operational period, based on a sixth time difference value (C6) between a second sensing time (vLS_SUP) and a start time (Br_SUP) of the far-field R-wave within the set up period, wherein the second sensing time is a sensing time corresponding to a sensed event in a first in-vivo near-field cardiac electrogram acquired based on the first electrode pair within a set up period, and the sensed event in the first in-vivo near-field cardiac electrogram is a near-field R-wave; and
within the operational period, estimating the start time (Br_OP) of the far-field R-wave within the operational period based on the fourth sensing time (aLS_OP), the time interval (PR_OP) between the near-field P-wave and the near-field R-wave within the operational period, and the sixth time difference value (C6).

Optionally, the control device is further configured to perform the following steps:
within a set up period, obtaining an eighth time difference value (C8) between a second sensing time (vLS_SUP) and a sensing time (GS_SUP) of a far-field R-wave within the set up period by the first electrode pair, wherein the second sensing time (vLS_SUP) is a sensing time corresponding to a sensed event in the first in-vivo near-field cardiac electrogram acquired by the first electrode pair within the set up period, and the sensed event in the first in-vivo near-field cardiac electrogram is a near-field R-wave; and
within the operational period, estimating the sensing time (GS_OP) of the far-field R-wave within the operational period based on the fourth sensing time (aLS_OP), a difference (PR_OP) between the first sensing time (vLS_OP) and the fourth sensing time (aLS_OP), and the eighth time difference value (C8).

Optionally, the control device is further configured to perform, during the operational period, the following step:
estimating the start time (Bp_OP) of the far-field P-wave within the operational period based on the fourth sensing time (aLS_OP), a start time (Bp_SUP) of the far-field P-wave within a set up period and a fifth sensing time (aLS_SUP);
where the fifth sensing time (aLS_SUP) is a sensing time corresponding to a sensed event in a second in-vivo near-field cardiac electrogram acquired by the second electrode pair within the set up period.

Optionally, the control device is further configured to perform the following step:
within an operational period, estimating the start time (Br_OP) or the sensing time (GS_OP) of the far-field R-wave within the operational period based on a difference value between the first sensing time (vLS_OP) and a first specific constant, the first specific constant being in the range of 0-150ms.

Optionally, the electrode pair is a second electrode pair, the cardiac electrical sensing time is a fourth sensing time (aLS_OP), the in-vivo near-field cardiac electrogram is a second in-vivo near-field cardiac electrogram, and the sensed event is a near-field P-wave.

Optionally, the control device is further configured to perform the following steps:
within a set up period, acquiring a start time (Br_SUP) of a far-field R-wave within the set up period; and
acquiring a start time (Bp_SUP) of a far-field P-wave within the set up period;
within the set up period or the operational period, acquiring a time interval (PR_SUP) between the far-field R-wave and the far-field P-wave within the set up period based on a difference (PR_SUP) between the start time (Br_SUP) of the far-field R-wave within the set up period and the start time (Bp_SUP) of the far-field P-wave within the set up period; and
within the operational period, estimating the start time (Br_OP) or the sensing time (GS_OP) of the far-field R-wave within the operational period based on the fourth sensing time (aLS_OP) and the time interval (PR_SUP) between the far-field R-wave and the far-field P-wave within the set up period.

Optionally, the control device is further configured to perform the following steps:
within the set up period or the operational period, acquiring an eleventh time difference value (C11) between a second sensing time (vLS_SUP) and a sensing time (GS_SUP) of the far-field R-wave within the set up period; where the second sensing time (vLS_SUP) is a sensing time corresponding to a sensed event in a first in-vivo near-field cardiac electrogram acquired by the first electrode pair within the set up period, and the sensed event in the first in-vivo near-field cardiac electrogram is a near-field R-wave; obtaining a twelfth constant (C12) based on a fifth sensing time (aLS_SUP), the time interval (PR_SUP) between the far-field R-wave and the far-field P-wave within the set up period and the eleventh time difference value (C11); and
estimating the sensing time (GS_OP) of the far-field R-wave within the operational period based on the fourth sensing time (aLS_OP) and the twelfth constant (C12) within the operational period.

Optionally, the control device is further configured to perform the following steps:
within the set up period or the operational period, obtaining a thirteenth time difference value (C13) between the second sensing time (vLS_SUP) and the start time (Br_SUP) of the far-field R-wave within the set up period; where the second sensing time (vLS_SUP) is a sensing time corresponding to a sensed event in a first in-vivo near-field cardiac electrogram acquired by the first electrode pair within the set up period, and the sensed event in the first in-vivo near-field cardiac electrogram is a near-field R-wave; obtaining a fourteenth constant (C14) based on the time interval (PR_SUP) between the far-field R-wave and the far-field P-wave within the set up period and the thirteenth time difference value (C13); and
within the operational period, estimating the start time (Br_OP) of the far-field R-wave within the operational period based on the fourth sensing time (aLS_OP) and the fourteenth constant (C14).

Optionally, the control device is further configured to perform the following step: within an operational period, estimating the start time (Br_OP) or the sensing time (GS_OP) of the far-field R-wave within the operational period based on a difference value between the fourth sensing time (aLS_OP) and a second specific constant, the second specific constant being in the range of 0-350 ms.

Optionally, a heartbeat generated during the operational period and a heartbeat generated during the set up period result from the same atrial or ventricular electrical activity; where the atrial or ventricular electrical activity is any one of: a sinus heartbeat, a ventricular heartbeat resulting from atrial conduction, a ventricular heartbeat resulting from ventricular pacing, and a ventricular heartbeat resulting from atrial pacing.

Optionally, the control device is further configured to perform, during the operational period, the following steps:
estimating a sensing time window corresponding to a far-field R-wave based on the estimated start time (Br_OP) or sensing time (GS_OP) of the far-field R-wave within the operational period; and
determining whether to deliver a CCM pulse based on the sensing time window and the cardiac electrical sensing time.

Optionally, the control device is further configured to perform, during the operational period, the following steps:
estimating a pulse delivery time window corresponding to a far-field R-wave based on the estimated start time (Br_OP) or sensing time (GS_OP) of the far-field R-wave within the operational period; and
determining whether to deliver a CCM pulse based on the pulse delivery time window and the cardiac electrical sensing time.

Optionally, the medical instrument further includes at least one ventricular electrode lead configured with the electrode pair for sensing, the electrode pair being a first electrode pair; or further includes at least one atrial electrode lead configured with the electrode pair for sensing, the electrode pair being a second electrode pair.

Optionally, the electrode pair is a first electrode pair or a second electrode pair for attachment to a ventricle or an epicardium of the ventricle.

By conforming to the common knowledge in the art, the optional conditions may be combined arbitrarily to obtain the embodiments of the present application.

The following are the positive and improved effects of the present application. The medical instrument according to the present application may estimate the start time or the sensing time of the far-field R-wave within the operational period, such that cardiac therapy, such as pacing and CCM, may be provided to the patient at proper time, thereby improving the efficacy of the medical instrument and the safety of the patient. Since it is no longer essential to depend on a real-time in-vivo far-field myocardial electrogram or in-vitro far-field cardiac electrogram, the structure of the medical instrument can be simplified, thereby reducing the energy consumption. In addition, even if the patient obtains an in-vivo far-field myocardial electrogram or an in-vitro far-field cardiac electrogram from an associated apparatus, the apparatus can quickly use the present application to determine the start time of the far-field R-wave in the event of any problem with the apparatus, to ensure normal operations of the apparatus. Further, any existing pacemaker can also be used in combination with the medical instrument according to the present application to provide CCM therapy, effectively reducing the patient's physical and economic burden.

### BRIEF DESCRIPTION OF THE DRAWINGS

Fig. 1 shows a schematic representation of a far-field cardiac electrogram, near-field atrial electrogram, and near-field ventricular electrogram.
Fig. 2 shows a flowchart of a method for controlling a medical instrument according to an embodiment of the present application.
Fig. 3 shows a schematic representation of a control device connected to an electrode pair according to an embodiment of the present application.
Fig. 4 shows a flowchart of a method for controlling a medical instrument according to Embodiment 1 of the present application.
Fig. 5 shows a schematic representation of waveforms of a far-field cardiac electrogram and a near-field cardiac electrogram for a set up period and an operational period.
Fig. 6 shows a flowchart of a method for controlling a medical instrument according to Embodiment 2 of the present application.
Fig. 7 shows a flowchart of a method for controlling a medical instrument according to Embodiment 3 of the present application.
Fig. 8 shows a schematic representation of other waveforms of the far-field cardiac electrogram and the near-field cardiac electrogram for the set up period and the operational period.
Fig. 9 shows a flowchart of a method for controlling a medical instrument according to Embodiment 4 of the present application.

### DETAILED DESCRIPTION OF THE PREFERRED EMBODIMENT

The present application is further illustrated by way of embodiment below, but is not thereby limited to the scope of the described embodiments.

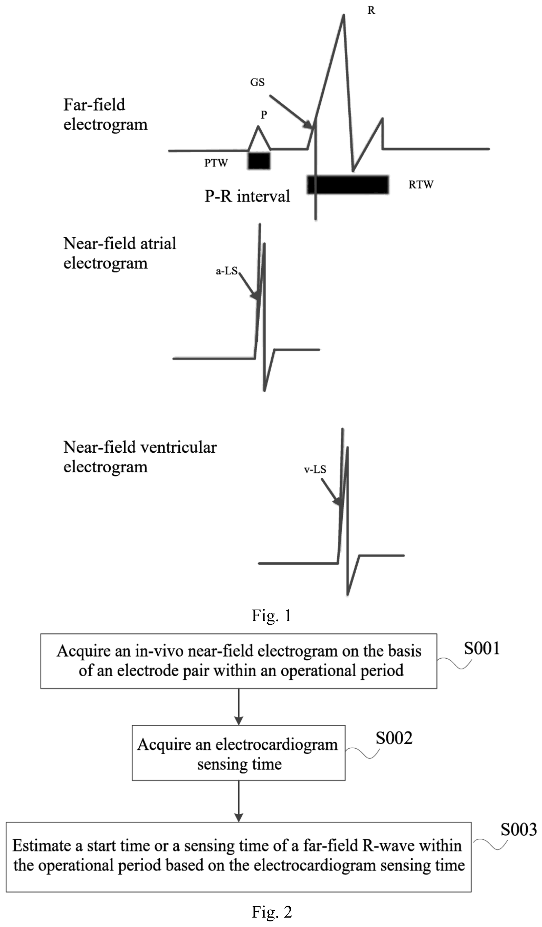

As shown in Fig. 1, a far-field cardiac electrogram (FF-ECG) is used to sense the overall electrical activity of the heart, and a P-wave time window (PTW) corresponds to an atrial activation window seen in the far-field cardiac electrogram and features its start time and duration, which means that all atrial activation signals measured at regional myocardia of various parts of the atria should fall within the PTW. It can be seen that an atrial activation signal a-LS in a near-field atrial electrogram in Fig. 1 falls within the PTW. Similarly, a window corresponding to the absolute refractory period of the entire atrial myocardium can be characterized by a start time and duration using a similar method. The R-wave time window (RTW) corresponds to the time window of the overall ventricular activation seen in the far-field cardiac electrogram, and features its start time and duration. This means that all ventricular activation signals measured at various regional myocardia of the ventricle should fall within the RTW. It can be seen that a ventricular activation signal v-LS in a near-field ventricular electrogram in Fig. 1 falls within the RTW. Similarly, a window corresponding to the absolute refractory period of the entire ventricular myocardium can be characterized by a start time and duration using a similar method.

It should be noted that the medical instrument according to the embodiments of the present application may particularly be an implantable single-chamber pacemaker, a dual-chamber pacemaker, a single-chamber implantable cardioverter defibrillator (ICD), a dual-chamber ICD, a cardiac resynchronization therapy defibrillator (CRT-D), a cardiac resynchronization therapy pacemaker (CRT-P), CCM, etc., or a temporary or long-term in-vitro medical instrument having similar single and/or combined functions.

A set up period (SUP) mentioned in the embodiments of the present application may be a period during which the medical instrument does not actually operate. For example, during the process of a surgeon implanting a medical instrument for a patient, the set up period mentioned in the embodiments of the present application may also be a period during which the medical instrument actually operates. For example, the set up period is a period of time for which the patient is hospitalized, and within which a surface far-field cardiac electrogram or an in-vivo far-field cardiac electrogram of the patient can be obtained by surface electrodes or temporary intracardiac electrode leads. For another example, the set up period is a period of time prior to an operational period, during which an actual surface far-field cardiac electrogram or in-vivo far-field cardiac electrogram is obtained using electrodes and associated devices. The operational period (OP) is a period during which the medical instrument actually operates. Parameters (such as sensing time, start time of a far-field R-wave) obtained within the set up period in the embodiments of the present application may be calculated values over a plurality of cardiac cycles under a certain relatively stable cardiac rhythm, such as a mean, a median, or another statistical value. Optionally, data within the set up period may also be updated and used within the operational period.

Furthermore, the far-field P-wave and R-wave mentioned in the embodiments of the present application is a short term for a P-wave and an R-wave in a far-field cardiac electrogram, which refer generally to electrical signals recorded in a far-field during the depolarization of the ventricular myocardium and correspond to a P-wave and an R-wave in an in-vivo (e.g., intracardiac or epicardial) far-field myocardial electrogram (EGM) or to a P-wave and a QRS-wave in a surface electrocardiogram (ECG). R-waves include various forms of QRS-waves, as long as they are medically considered as QRS-waves.

The medical instrument according to the embodiments of the present application may estimate the start time or the sensing time of a far-field R-wave within the operational period, be part of the determination of sensing, and allow cardiac therapy, such as pacing and CCM, to be provided to the patient at proper time, thus improving the efficacy of the medical instrument and the safety of the patient.

In the present application, for sensing information acquired by electrode pairs located at the same position within the operational period and the set up period, near-field R-waves, near-field P-waves, far-field R-waves and far-field P-waves within the operational period have an equivalent time interval relationship with near-field R-waves, near-field P-waves, far-field R-waves and far-field P-waves within the set up period by default.

The present application provides a medical instrument, including a control device. As shown in Fig. 2, the control device is configured to perform the following steps S001-S003.

In step S001, an in-vivo near-field cardiac electrogram is acquired by an electrode pair within an operational period. The in-vivo near-field cardiac electrogram is also known as L-EGM (local electrogram, and sometimes is also known as near-field electrogram (NF-EGM)).

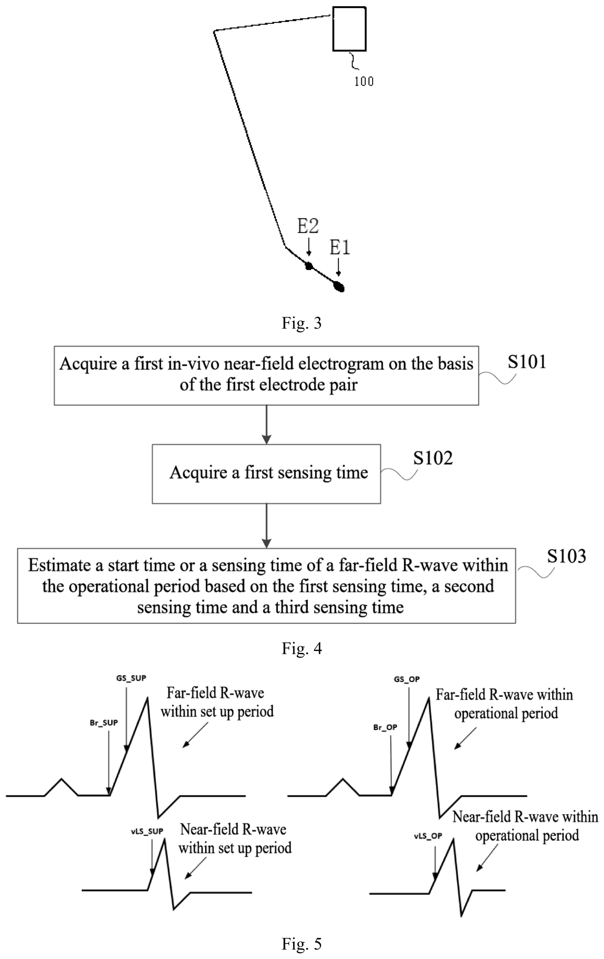

Fig. 3 shows a schematic representation of a control device connected to an electrode pair. As shown in Fig. 3, the control device 100 is connected to the electrode pair, and the control device 100 acquires the in-vivo near-field cardiac electrogram by the electrode pair. The electrode pair includes an electrode E1 and an electrode E2.

In step S002, a cardiac electrical sensing time is acquired; where the cardiac electrical sensing time is a sensing time corresponding to a sensed event in the in-vivo near-field cardiac electrogram.

In step S003, a start time or a sensing time of a far-field R-wave within the operational period is estimated based on the cardiac electrical sensing time.

In certain embodiments of the present application, the electrode pair is a first electrode pair, the cardiac electrical sensing time is a first sensing time, the in-vivo near-field cardiac electrogram is a first in-vivo near-field cardiac electrogram, the sensed event is a near-field R-wave, and the start time or sensing time of the far-field R-wave within the operational period can be estimated by acquiring the sensing time of the near-field R-wave in the first in-vivo near-field cardiac electrogram by the first electrode pair.

In some other embodiments of the present application, the electrode pair is a second electrode pair, the cardiac electrical sensing time is a fourth sensing time, the in-vivo near-field cardiac electrogram is a second in-vivo near-field cardiac electrogram, the sensed event is a near-field P-wave, and the start time or sensing time of the far-field R-wave within the operational period can be estimated by acquiring the sensing time of the near-field P-wave in the second in-vivo near-field cardiac electrogram by the second electrode pair.

In some other embodiments of the present application, the medical instrument further includes at least one ventricular electrode lead configured with the electrode pair for sensing, the electrode pair being a first electrode pair. Alternatively, the medical instrument further includes at least one atrial electrode lead configured with the electrode pair for sensing, the electrode pair being a second electrode pair.

In some other embodiments of the present application, the electrode pair is a first electrode pair or a second electrode pair for attachment to a ventricle or an epicardium of the ventricle.

### Embodiment 1

Fig. 4 shows a schematic flowchart of a method for controlling a medical instrument according to this embodiment. The control method can be performed by a control device of the medical instrument during the operational period or during the set up period and the operational period. The control device can be implemented in software and hardware. The control device may be a part of the medical instrument. The control device can be used in vitro or implanted in a human body.

As shown in Fig. 4, the method for controlling a medical instrument according to this embodiment may include the following steps S101-S103.

In step S101, a first in-vivo near-field cardiac electrogram is acquired by a first electrode pair.

In step S102, a first sensing time is acquired, where the first sensing time is a sensing time corresponding to a sensed event in the first in-vivo near-field cardiac electrogram, and the sensed event is a near-field R-wave.

In step S103, a start time or a sensing time of a far-field R-wave within the operational period is estimated based on the first sensing time, a second sensing time and a third sensing time.

The second sensing time is a sensing time corresponding to a sensed event in a first in-vivo near-field cardiac electrogram acquired by the first electrode pair within a set up period, and the third sensing time is a sensing time of an R-wave in a far-field cardiac electrogram acquired within a set up period. The far-field cardiac electrogram can also be referred to as FF-ECG or FF-EGM (far-field electrogram). Steps S101-S103 may all be performed within the operational period, or only steps S101-S102 are performed within the operational period, and step S103 is performed in different period of time within the set up period and the operational period.

As shown in Fig. 5, it is assumed that the first sensing time acquired within the operational period is vLS_OP, the second sensing time acquired within the set up period is vLS_SUP, the third sensing time acquired within the set up period is GS_SUP (GS = global sensing), and the start time of the far-field R-wave within the operational period is Br_OP, the start time Br_OP of the far-field R-wave is estimated based on the first sensing time vLS_OP, the second sensing time vLS_SUP and the third sensing time GS_SUP in this embodiment. A first time difference value between the second sensing time vLS_SUP within the set up period and the start time Br_SUP of the far-field R-wave within the set up period is equal to a second time difference value between the first sensing time vLS_OP within the operational period and the start time Br_OP of the far-field R-wave within the operational period by default. In a specific example, Br_OP = vLS_OP- (vLS_SUP-Br_SUP) = vLS_OP- (vLS_SUP- (GS_SUP-C1)). C1 is a first constant, which can be customized to be in the range of 0-100 ms, preferably 0-50 ms, according to actual situations. For example, C1 can be set to 20 ms. In this case, the first constant C1 is an empirical difference value between the sensing time of the R-wave and the start time. In another implementation, the control device is further configured to perform, during the set up period, the following step: obtaining a first time difference value between a third sensing time GS_SUP and the start time Br_SUP of the far-field R-wave within the operational period, and obtaining the first constant C1 based on the first time difference value, for example, the first time difference value serving as the first constant C1, whereby the first constant C1 is more specific to the patient, and the first time difference value can also be manually input into the control device by a surgeon.

Further, Br_OP = vLS_OP- (vLS_SUP- (GS_SUP-C1) = vLS_OP-C2. C2 is a second constant, the second constant C2 can be obtained from a second time difference value between the second sensing time vLS_SUP acquired within the set up period and a difference between the third sensing time GS_SUP and C1, i.e., C2 = vLS_SUP- (GS_SUP-C1), and the start time Br_OP of the far-field R-wave within the operational period can be estimated in real time based on the first sensing time vLS_OP obtained in real time within the operational period and the constant C2. In an implementation, the above step of calculating the second constant C2 may be performed within the set up period. After the second constant C2 is obtained, the second constant C2 obtained within the set up period may be saved in a memory of the control device for calling within the operational period, to estimate the start time Br_OP of the R-wave in the far-field cardiac electrogram. In another implementation, the vLS_OP, vLS_SUP, GS_SUP and C1 obtained within the set up period can be directly saved in the memory, and the above steps are performed within the operational period to estimate the start time Br_OP of the R-wave in the far-field cardiac electrogram.

In an implementation, a first specific constant may be preset in the memory, without going through the set up period. Within the operational period, the start time (Br_OP) or sensing time (GS_OP) of the far-field R-wave within the operational period can be estimated directly from a difference value between the first sensing time (vLS_OP) and the first specific constant. The first specific constant is in the range of 0-150 ms, or preferably 0-50 ms. In this implementation, for example, the second constant C2 may serve as the first specific constant. It may be understood that in this implementation, a constant range can also be preset in the memory, the first specific constant can be manually selected by the surgeon from the constant range, and the present invention is not limited to this.

It may be understood that in Fig. 5, the far-field R-wave includes only an R-band and a P-band. In other embodiments, the far-field R-wave may also include a Q-band, that is, the far-field R-wave is a complete QRS-wave, and in this case, the start time of the far-field R-wave may also be a start time of the Q-band.

It can be seen from the above embodiment that, according to the present application, no far-field cardiac electrogram is required for the operational period, and that the start time Br_OP of the far-field R-wave within the operational period can be estimated in real time with only the first sensing time vLS_OP of the near-field myocardial electrogram.

In an optional implementation, the above control method further includes estimating a sensing time of an R-wave in a far-field cardiac electrogram corresponding to the operational period from the first sensing time, the second sensing time and the third sensing time.

It is assumed that the sensing time of the R-wave in the far-field cardiac electrogram corresponding to the operational period is GS_OP, GS_OP is estimated from vLS_OP, vLS_SUP and GS_SUP in this embodiment. In a specific example, GS_OP = vLS_OP-(vLS_SUP-GS_SUP) = vLS_OP-C3. C3 is a third constant, and particularly a third time difference value between the second sensing time vLS_SUP and the third sensing time GS_SUP obtained within the set up period. The sensing time GS_OP of the R-wave in the far-field cardiac electrogram corresponding to the operational period can be estimated in real time based on the first sensing time vLS_OP obtained in real time within the operational period and the third constant C3. It may be understood that the step of calculating the third constant C3 may be performed during the set up period, and the third constant C3 is saved in the memory of the control device. The second sensing time vLS_SUP and the third sensing time GS_SUP may also be saved in the memory, and during the operational period, the first sensing time vLS_OP, the second sensing time vLS_SUP and the third sensing time GS_SUP are used to estimate the sensing time GS_OP of the R-wave in the far-field cardiac electrogram within the operational period. Further, when the estimated sensing time GS_OP of the R-wave in the far-field cardiac electrogram is obtained, the start time Br_OP of the R-wave in the far-field cardiac electrogram can also be estimated, in particular by Br_OP = GS_OP-C1. The method for obtaining C1 has been described in the previous implementation, and will not be repeated herein. The third constant C3 may also serve as the first specific constant, and the method for setting the first specific constant has been described in the previous implementation and will not be repeated herein.

According to the above embodiment, it can be appreciated that in the present application, the start time of the R-wave in the far-field cardiac electrogram within the operational period can be estimated from the first sensing time, the second sensing time and the third sensing time; the sensing time of the R-wave in the far-field cardiac electrogram within the operational period can also be estimated from the first sensing time, the second sensing time and the third sensing time; and the start time of the R-wave in the far-field cardiac electrogram within the operational period can also be further estimated after the sensing time of the R-wave in the far-field cardiac electrogram within the operational period has been estimated.

### Embodiment 2

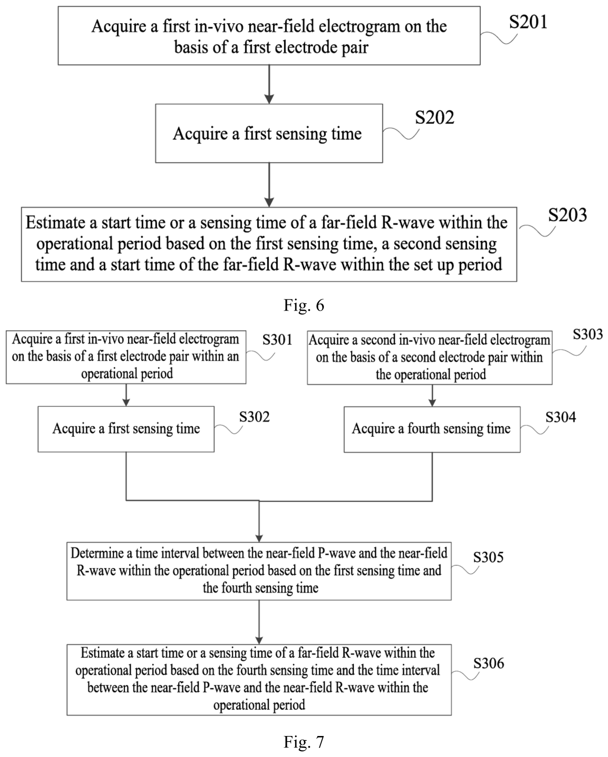

Fig. 6 shows a schematic flowchart of a method for controlling a medical instrument according to this embodiment. The control method can be performed by a control device of the medical instrument during the operational period or during the set up period and the operational period. The control device can be implemented in software and hardware. The control device may be a part of the medical instrument. The control device can be used in vitro or implanted in a human body.

As shown in Fig. 6, the method for controlling a medical instrument according to this embodiment may include the following steps S201-S203.

In step S201, a first in-vivo near-field cardiac electrogram is acquired by a first electrode pair.

In step S202, a first sensing time is acquired, where the first sensing time is a sensing time corresponding to a sensed event in the first in-vivo near-field cardiac electrogram, and the sensed event is a near-field R-wave.

In step S203, a start time or a sensing time of a far-field R-wave within the operational period is estimated based on the first sensing time, a second sensing time and a start time of the far-field R-wave within the set up period.

The second sensing time is a sensing time corresponding to a sensed event in the first in-vivo near-field cardiac electrogram acquired by the first electrode pair within the set up period.

Referring also to Fig. 5, it is assumed that the first sensing time acquired within the operational period is vLS_OP, the second sensing time acquired within the set up period is vLS_SUP, the start time of the far-field R-wave within the set up period is Br_SUP, and the start time of the far-field R-wave within the operational period is Br_OP, Br_OP is estimated based on vLS_OP, vLS_SUP, and Br_SUP in this embodiment. In a specific example, Br_OP = vLS_OP- (vLS_SUP-Br_SUP) = vLS_OP-C4. C4 is a fourth constant, and particularly a fourth time difference value between the second sensing time vLS_SUP obtained within the set up period and the start time Br_SUP of the far-field R-wave, i.e., C4 = vLS_SUP-Br_SUP, and the start time Br_OP of the far-field R-wave within the operational period can be estimated in real time based on the first sensing time vLS_OP obtained in real time within the operational period and the constant C4, that is, no far-field cardiac electrogram is required within the operational period, and only the first sensing time vLS_OP of a near-field myocardial electrogram is required to estimate the start time of the far-field R-wave within the operational period. It may be understood that the step of calculating the fourth constant may be performed within a preset value, the obtained fourth constant is saved in the memory of the control device, or the second sensing time vLS_SUP and the start time Br_SUP of the far-field R-wave within the set up period are saved in the memory first, and the start time Br_OP of the far-field R-wave within the operational period is estimated directly within the operational period. The fourth constant C4 may also serve as the first specific constant, and the method for setting the first specific constant has been described in the previous embodiment and will not be repeated herein.

It should be noted that the start time Br_SUP of the far-field R-wave within the set up period can be estimated by other methods.

In an optional implementation, the start time of the far-field R-wave within the set up period is determined based on the sensing time of the R-wave in the far-field cardiac electrogram acquired within the set up period.

It is assumed that the sensing time of the R-wave in the far-field cardiac electrogram acquired within the set up period is GS_SUP, the start time Br_SUP of the far-field R-wave within the set up period is determined based on GS_SUP in this implementation. In a specific example, Br_SUP = GS_SUP-C5. C5 is a constant, which can be customized according to actual situations, and C5 can be in the range of 0-100 ms, or preferably 0-50 ms. For example, C5 can be set to 20 ms.

### Embodiment 3

Fig. 7 shows a schematic flowchart of a method for controlling a medical instrument according to this embodiment. The control method can be performed by a control device of the medical instrument during the operational period or during the set up period and the operational period. The control device can be implemented in software and hardware. The control device may be a part of the medical instrument. The control device can be used in vitro or implanted in a human body.

As shown in Fig. 7, the method for controlling a medical instrument according to this embodiment may include the following steps S301-S306.

In step S301, a first in-vivo near-field cardiac electrogram is acquired by a first electrode pair within an operational period.

In step S302, a first sensing time is acquired, where the first sensing time is a sensing time corresponding to a sensed event in the first in-vivo near-field cardiac electrogram, and the sensed event is a near-field R-wave.

In step S303, a second in-vivo near-field cardiac electrogram is acquired by a second electrode pair within the operational period.

In step S304, a fourth sensing time is acquired, where the fourth sensing time is a sensing time corresponding to a sensed event in the second in-vivo near-field cardiac electrogram, and the sensed event is a near-field P-wave.

In step S305, a time interval between the near-field P-wave and the near-field R-wave within the operational period is determined based on the first sensing time and the fourth sensing time.

In step S306, a start time or a sensing time of a far-field R-wave within the operational period is estimated based on the fourth sensing time and the time interval between the near-field P-wave and the near-field R-wave within the operational period.

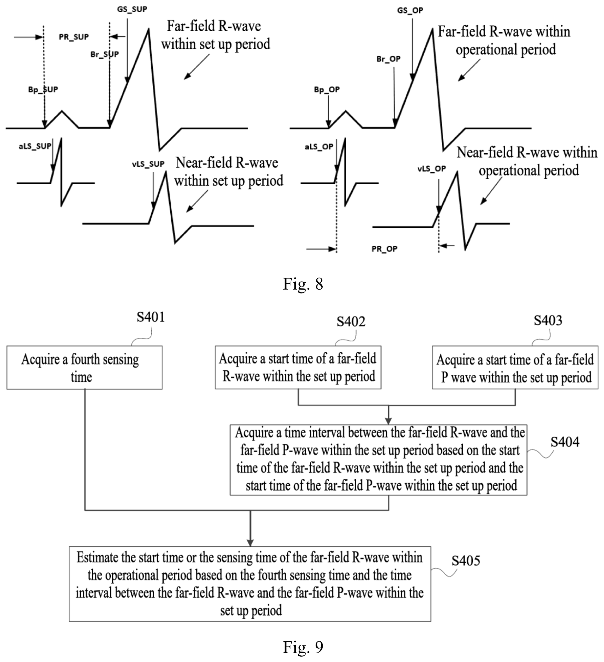

As shown in Fig. 8, it is assumed that the first sensing time acquired within the operational period is vLS_OP and the fourth sensing time is aLS_OP, a difference between the first sensing time vLS_OP and the fourth sensing time aLS_OP may be the time interval PR_OP between the near-field P-wave and the near-field R-wave within the operational period, i.e., PR_OP = vLS_OP-aLS_OP. It is assumed that the start time of the far-field R-wave within the operational period is Br_OP, Br_OP is estimated based on the fourth sensing time aLS_OP and the time interval PR_OP in this embodiment. In a specific example, Br_OP = aLS_OP+ (PR_OP-C6) = aLS_OP+C7. C6 is a sixth constant, which can be set according to actual situations. Specifically, a sixth time difference value between the second sensing time vLS_SUP acquired within the set up period and the start time Br_SUP of the far-field R-wave within the set up period can be obtained within the set up period, and this sixth time difference value serves as the sixth constant C6 within the operational period. C7 can be obtained from a difference value between the time interval PR_OP between the near-field P-wave and the near-field R-wave within the operational period and C6, and the start time Br_OP of the far-field R-wave within the operational period can be estimated in real time based on the fourth sensing time aLS_OP obtained in real time within the operational period and C7. Like the other embodiments described above, the step of calculating the sixth time difference value may be performed within the set up period or within the operational period.

In an implementation, a second specific constant may be preset in the memory, without going through the set up period. Within the operational period, the start time (Br_OP) or sensing time (GS_OP) of the far-field R-wave within the operational period can be estimated directly from a difference value between the fourth sensing time (aLS_OP) and the second specific constant. The second specific constant is in the range of 0-350 ms, or preferably 0-50 ms. In this implementation, for example, the seventh constant C7 may serve as the second specific constant. It may be understood that in this implementation, a constant range can also be preset in the memory, the first specific constant can be manually selected by the surgeon from the constant range, and the present invention is not limited to this.

In another implementation, it is assumed that the sensing time of the far-field R-wave within the operational period is GS_OP, the sensing time GS_OP of the far-field R-wave within the operational period is estimated based on the fourth sensing time aLS_OP and the time interval PR_OP. In a specific example, GS_OP = aLS_OP+ (PR_OP - C8) = aLS_OP+C9. C8 is a constant, which can be set according to actual situations. Specifically, an eighth time difference value between the second sensing time vLS_SUP acquired within the set up period and the sensing time GS_SUP of the far-field R-wave within the set up period can be obtained within the set up period, i.e., vLS_SUP-GS_SUP. Within the operational period, this eighth time difference value serves as the constant C8, and a ninth constant C9 can be obtained from a ninth time difference value between the time interval PR_OP and C8. The sensing time GS_OP of the far-field R-wave within the operational period can be estimated in real time based on the fourth sensing time aLS_OP obtained in real time within the operational period and C9. In an implementation, the ninth constant C9 may also serve as the second specific constant, and the method for setting the second specific constant has been described in the previous implementation and will not be repeated herein.

That is, in this embodiment, no far-field cardiac electrogram is required for the operational period, and only the first sensing time vLS_OP and the fourth sensing time aLS_OP of the near-field myocardial electrogram are required to estimate the start time or the sensing time of the far-field R-wave within the operational period.

In an optional implementation, the above control method further includes estimating the start time Bp_OP of the far-field P-wave within the operational period based on the fourth sensing time aLS_OP, a start time Bp_SUP of the far-field P-wave within the set up period and a fifth sensing time aLS_SUP. The fifth sensing time is a sensing time (aLS_SUP) corresponding to a sensed event in the second in-vivo near-field cardiac electrogram acquired by the second electrode pair within the set up period.

In an optional implementation, as shown in Fig. 8, it is assumed that the start time of the far-field P-wave within the set up period is Bp_SUP and the fifth sensing time is aLS_SUP, and the start time of the far-field P-wave within the operational period is Bp_OP. In this embodiment, the start time Bp_OP of the far-field P-wave within the operational period is estimated based on the fourth sensing time aLS_OP, the start time Bp_SUP of the far-field P-wave within the set up period and the fifth sensing time aLS_SUP. A time difference value between aLS_OP and Bp_OP is equal to a time difference value between aLS_SUP and Bp_SUP by default. In a specific example, Bp_OP = aLS_OP+(Bp_SUP- aLS_SUP) =aLS_OP+C10. C10 is a constant, and particularly a tenth time difference value between the start time Bp_SUP of the far-field P-wave and the fifth sensing time aLS_SUP obtained within the set up period. The start time Bp_OP of the far-field P-wave within the operational period can be estimated in real time based on the fourth sensing time aLS_OP obtained in real time within the operational period and the constant C10. The tenth constant C10 may also serve as the second specific constant, and the method for setting the second specific constant has been described in the previous implementation and will not be repeated herein.

### Embodiment 4

Fig. 9 shows a schematic flowchart of a method for controlling a medical instrument according to this embodiment. The control method can be performed by a control device of the medical instrument during the operational period or during the set up period and the operational period. The control device can be implemented in software and hardware. The control device may be a part of the medical instrument. The control device can be used in vitro or implanted in a human body.

As shown in Fig. 9, the method for controlling a medical instrument according to this embodiment may include the following steps S401-S405.

In step S401, a fourth sensing time aLS_OP is acquired, where the fourth sensing time is a sensing time corresponding to a sensed event in a second in-vivo near-field cardiac electrogram acquired by a second electrode pair within the operational period.

In step S402, within the set up period, a start time Br_SUP of a far-field R-wave within the set up period is acquired.

In step S403, within the set up period, a start time Bp_SUP of a far-field P-wave within the set up period is acquired.

In step S404, within the set up period or the operational period, a time interval PR_SUP between the far-field R-wave and the far-field P-wave within the set up period is acquired based on the start time Br_SUP of the far-field R-wave within the set up period and the start time Bp_SUP of the far-field P-wave within the set up period.

In step S405, within the operational period, the start time Br_OP or the sensing time GS_OP of the far-field R-wave within the operational period is estimated based on the fourth sensing time aLS_OP and the time interval PR_SUP between the far-field R-wave and the far-field P-wave within the set up period.

As shown in Fig. 8, in an optional implementation, the fourth sensing time aLS_OP is acquired by the second electrode pair within the operational period, the start time Br_SUP of the far-field R-wave within the set up period and the start time Bp_SUP of the far-field P-wave are acquired within the set up period, and a difference between the start time Br_SUP of the far-field R-wave and the start time Bp_SUP of the far-field P-wave serves as the time interval PR_SUP between the far-field P-wave and the far-field R-wave within the set up period, i.e., PR_SUP = Br_SUP-Bp_SUP. It is assumed that the start time of the far-field P-wave within the operational period is aLS_OP, GS_OP is estimated based on aLS_OP and PR_SUP in this embodiment. In a specific example, GS_OP = aLS_OP+ (PR_SUP - C11) = aLS_OP+C12. C11 is a constant, which can be set according to actual situations. Specifically, an eleventh time difference value between the second sensing time vLS_SUP acquired within the set up period and the sensing time GS_SUP of the far-field R-wave within the set up period can be obtained within the set up period, where the second sensing time vLS_SUP is a sensing time corresponding to a sensed event in the first in-vivo near-field cardiac electrogram acquired by the first electrode pair within the set up period. Within the operational period, the eleventh time difference value serves as the eleventh constant C11. A twelfth constant C12 can be obtained from a difference value between the time interval PR_SUP between the far-field P-wave and the far-field R-wave within the set up period and C11, that is, the twelfth constant C12 can be obtained from parameters for the set up period. During the operational period, the sensing time GS_OP of the far-field R-wave within the operational period can be estimated in real time based on the fourth sensing time aLS_OP.

In an implementation, the twelfth constant C12 may also serve as the second specific constant, and the method for setting the second specific constant has been described in the previous implementation and will not be repeated herein.

In another implementation, it is assumed that the fourth sensing time aLS_OP is acquired within the operational period, the start time Br_SUP of the far-field R-wave within the set up period and the start time Bp_SUP of the far-field P-wave are acquired within the set up period, and a difference between the start time Br_SUP of the far-field R-wave and the start time Bp_SUP of the far-field P-wave serves as the time interval PR_SUP between the far-field P-wave and the far-field R-wave within the set up period, i.e., PR_SUP = Br_SUP-Bp_SUP. It is assumed that the start time of the far-field P-wave within the operational period is aLS_OP, Br_OP is estimated based on aLS_OP and PR_SUP in this embodiment. In a specific example, Br_OP = aLS_OP+ (PR_SUP - C13) = aLS_OP+C14. C13 is a constant, which can be set according to actual situations. Specifically, a thirteenth time difference value between the second sensing time vLS_SUP acquired within the set up period and the start time Br_SUP of the far-field R-wave within the set up period can be obtained within the set up period, where the second sensing time vLS_SUP is a sensing time corresponding to a sensed event in the first in-vivo near-field cardiac electrogram acquired by the first electrode pair within the set up period. Within the operational period, the thirteenth time difference value serves as the thirteenth constant C13. A fourteenth constant C14 can be obtained from a difference value between the time interval PR_SUP between the far-field P-wave and the far-field R-wave within the set up period and C13, that is, the fourteenth constant C14 can be obtained from parameters for the set up period. During the operational period, the start time Br_OP of the far-field R-wave within the operational period can be estimated in real time based on the fourth sensing time aLS_OP.

In an implementation, the thirteenth constant C13 may also serve as the second specific constant, and the method for setting the second specific constant has been described in the previous implementation and will not be repeated herein.

It may be understood that, in the present application, the set up period and the operational period are provided, and based on various parameters within the set up period, including but not limited to aLS_SUP, vLS_SUP, Bp_SUP, Br_SUP, PR_SUP, etc., the start time Br_OP or the sensing time GS_OP of the far-field R-wave within an estimated operational period can be estimated by obtaining one or more of vLS_OP, aLS_OP and PR_OP within the operational period. The actual monitoring of a far-field cardiac electrogram can be dispensed with within the operational period by effectively using known information of the set up period. The medical instrument according to the present application allow to determine a time window for a CCM pulse covering the absolute refractory period of the entire ventricular myocardium with only one ventricular electrode lead, to achieve safe delivery of CCM pulse stimulations, regardless of the medical instrument being a single-chamber pacemaker, dual-chamber pacemaker or cardiac resynchronization pacemaker.

It should be noted that the following applies to the control method according to the foregoing Embodiments 1 to 4. In an optional implementation, a heartbeat generated during the operational period and a heartbeat generated during the set up period result from the same atrial or ventricular electrical activity. Specifically, the atrial or ventricular electrical activity may be a sinus heartbeat, a ventricular heartbeat resulting from atrial conduction, a ventricular heartbeat resulting from ventricular pacing, a ventricular heartbeat resulting from atrial pacing, etc.

In an optional implementation, the above control method further includes estimating a sensing time window corresponding to the R-wave in the far-field cardiac electrogram based on the estimated start time Br_OP or sensing time GS_OP of the far-field R-wave within the operational period, and determining whether to deliver a CCM pulse based on the sensing time window and the cardiac electrical sensing time. The sensing time window corresponds to the R-wave time window RTW described above.

In specific practice, a start point of the sensing time window can be estimated first based on the estimated start time Br_OP or sensing time GS_OP of the far-field R-wave within the operational period, and a length of the sensing time window is then determined based on the current ventricular electrical activity. In a specific example, if the current ventricular electrical activity is a ventricular heartbeat resulting from atrial conduction, a width of the R-wave in the far-field cardiac electrogram is normal, and typically less than 120 ms, the length of the sensing time window may be in the range of 30-160 ms, for example, the length of the sensing time window may be determined to be 100 ms. In another specific example, if the current ventricular electrical activity is a ventricular heartbeat resulting from ventricular pacing, the R-wave in the far-field cardiac electrogram is wide, and typically between 160 ms and 250 ms, and the length of the sensing time window can also be determined to be 200 ms. The ventricular electrical activity can be determined by a conventional method, such as a method for detection of premature ventricular contraction (PVC).

It should be noted that the length of the above sensing time window can also be obtained by other methods, for example by being directly programmed by an external device (such as a programmer) by the surgeon inputting the length of the sensing time window to modify a preset value.

In an optional implementation, the above control method further includes estimating a pulse delivery time window corresponding to the R-wave in the far-field cardiac electrogram based on the estimated start time Br_OP or sensing time GS_OP of the far-field R-wave within the operational period, and determining whether to deliver a CCM pulse based on the pulse delivery time window and the cardiac electrical sensing time.

The pulse delivery time window can be referred to as a CCM stimulation safety window. The pulse delivery time window corresponds to a safety period, which corresponds to a CCM pulse delivery period of the entire ventricle, aiming to cover the absolute refractory period of the entire ventricular myocardium. Specifically, a start point of the pulse delivery time window can be estimated based on the estimated start time Br_OP or sensing time GS_OP of the far-field R-wave within the operational period, and an end point thereof corresponds to or is slightly earlier than an end time point of the absolute refractory period of its ventricular myocardium. The length of the pulse delivery time window may be preset to 200 ms, and has a selectable range including, but are not limited to, 150-300 ms. Adaptive adjustments can be specifically made based on the patient's conditions (e.g., use or no use of antiarrhythmic drugs such as amiodarone, which may prolong the absolute refractory period of the myocardium), actual R-wave sensing, and other factors.

The start points of the above pulse delivery time window and the above sensing time window may be the same or different. The two time windows, i.e., the pulse delivery time window and the sensing time window, can be set independently and can be independent of each other.

The control device in the embodiments of the present application may be implemented in the form of software called by a processor. For example, the control device includes a processor and a memory, where the processor is connected to the memory, instructions and parameters are stored in the memory, including obtaining the parameters or preset parameters or a range of parameter within the set up period, and the processor calls the instructions and the parameters stored in the memory to implement the functions of a control unit. The processor is, for example, a general-purpose processor, such as a central processing unit, the memory may be integrated with or separate from the processor, for example an on-chip memory or an off-chip memory storing a computer program executable by the at least one processor. The computer program, when being executed by the at least one processor, causes the at least one processor to perform the control method according to this embodiment. Alternatively, the control unit may be implemented in the form of a hardware circuit, the function of which may be implemented by means of the design of the hardware circuit. The hardware circuit may be understood as one or more processors. For example, in an implementation, the hardware circuit is an application specific integrated circuit, and logical relationships between elements of the circuit are designed to implement the function of the above control unit. In the embodiments of the present application, the processor is a circuit with signal processing capability, and in an implementation, the processor may be an instruction-readable and executable circuit, for example a CPU, a microprocessor, or a digital signal processor, etc. In another implementation, the processor may implement a certain function by means of the logical relationship of the hardware circuit which is fixed or reconfigurable. For example, the processor is a hardware circuit implemented by an ASIC or PLD, such as field programmable gate array.

Although the specific implementations of the present application are described above, it should be appreciated by those skilled in the art that these are merely illustrative and that the scope of protection of the present application is defined by the appended claims. Various changes or modifications to these implementations may be made by those skilled in the art without departing from the principle and spirit of the present application, and these changes or modifications fall within the scope of the present application.

## Claims

1. A medical instrument, comprising a control device configured to perform the following steps:
acquiring an in-vivo near-field cardiac electrogram by an electrode pair within an operational period;
acquiring a cardiac electrical sensing time; wherein the cardiac electrical sensing time is a sensing time corresponding to a sensed event in the in-vivo near-field cardiac electrogram; and
estimating a start time (Br_OP) or a sensing time (GS_OP) of a far-field R-wave within the operational period based on the cardiac electrical sensing time.

2. The medical instrument of claim 1, wherein the electrode pair is a first electrode pair, the cardiac electrical sensing time is a first sensing time (vLS_OP), the in-vivo near-field cardiac electrogram is a first in-vivo near-field cardiac electrogram, and the sensed event is a near-field R-wave.

3. The medical instrument of claim 2, wherein
the control device is further configured to perform the following step:
estimating the start time (Br_OP) or the sensing time (GS_OP) of the far-field R-wave within the operational period based on the first sensing time (vLS_OP), a second sensing time (vLS_SUP) and a third sensing time (GS_SUP);
wherein the second sensing time (vLS_SUP) is a sensing time corresponding to a sensed event in a first in-vivo near-field cardiac electrogram acquired by the first electrode pair within a set up period, and the third sensing time (GS_SUP) is a sensing time of an R-wave in a far-field cardiac electrogram acquired within a set up period.

4. The medical instrument of claim 3, wherein the control device is further configured to perform the following steps:
within the set up period or the operational period, obtaining a second constant (C2) based on the second sensing time (vLS_SUP), the third sensing time (GS_SUP) and a first time difference value (C1) between the third sensing time (GS_SUP) and a start time (Br_SUP) of a far-field R-wave within the set up period; and
within the operational period, estimating the start time (Br_OP) of the R-wave in the far-field cardiac electrogram within the operational period based on the first sensing time (vLS_OP) and the second constant (C2).

5. The medical instrument of claim 3, wherein the control device is further configured to perform the following steps:
within the set up period or the operational period, obtaining a second constant (C2) based on the second sensing time (vLS_SUP), the third sensing time (GS_SUP) and a first constant (C1); and
within the operational period, estimating the start time (Br_OP) of the far-field R-wave within the operational period based on the first sensing time (vLS_OP) and the second constant (C2).

6. The medical instrument of claim 2, wherein the control device is further configured to perform the following steps:
obtaining a third time difference value (C3) between a second sensing time (vLS_SUP) and a third sensing time (GS_SUP) within a set up period or the operational period, wherein the second sensing time (vLS_SUP) is a sensing time corresponding to a sensed event in a first in-vivo near-field cardiac electrogram acquired by the first electrode pair within the set up period, and the third sensing time (GS_SUP) is a sensing time of an R-wave in a far-field cardiac electrogram acquired within the set up period; and
estimating the sensing time (GS_OP) of the far-field R-wave within the operational period based on the first sensing time (vLS_OP) and the third time difference value (C3) within the operational period.

7. The medical instrument of claim 6, wherein the control device is further configured to perform, during the operational period, the following step:
within the operational period, estimating the start time (Br_OP) of the R-wave in the far-field cardiac electrogram within the operational period based on the estimated sensing time (GS_OP) of the R-wave in the far-field cardiac electrogram within the operational period and a first constant (C1), wherein the first constant is in the range of 0-100 ms.

8. The medical instrument of claim 2, wherein the control device is further configured to perform the following step:
estimating the start time (Br_OP) or the sensing time (GS_OP) of the far-field R-wave within the operational period based on the first sensing time (vLS_OP), a second sensing time (vLS_SUP) and a start time (Br_SUP) of a far-field R-wave within a set up period;
wherein the second sensing time is a sensing time corresponding to a sensed event in a first in-vivo near-field cardiac electrogram acquired by the first electrode pair within a set up period.

9. The medical instrument of claim 2, wherein the control device is further configured to perform the following steps:
within a set up period or the operational period, obtaining a fourth time difference value (C4) between a second sensing time (vLS_SUP) and a start time (Br_SUP) of a far-field R-wave within the set up period; wherein the second sensing time (vLS_SUP) is a sensing time corresponding to a sensed event in the first in-vivo near-field cardiac electrogram acquired by of the first electrode pair within the set up period; and
within the operational period, estimating the start time (Br_OP) of the far-field R-wave within the operational period based on the first sensing time (vLS_OP) and the fourth time difference value (C4).

10. The medical instrument of claim 1, wherein the electrode pair is a second electrode pair, the cardiac electrical sensing time is a fourth sensing time (aLS_OP), the in-vivo near-field cardiac electrogram is a second in-vivo near-field cardiac electrogram, and the sensed event is a near-field P-wave; and
wherein the control device is further configured to perform the following steps:
acquiring a first sensing time (vLS_OP); wherein the first sensing time (vLS_OP) is a sensing time corresponding to a sensed event in a first in-vivo near-field cardiac electrogram acquired by a first electrode pair within an operational period;
determining a time interval (PR_OP) between a near-field P-wave and a near-field R-wave within the operational period based on a first sensing time (vLS_OP) and the fourth sensing time (aLS_OP); and
estimating the start time (Br_OP) or the sensing time (GS_OP) of the far-field R-wave within the operational period based on the fourth sensing time (aLS_OP) and the time interval (PR_OP) between the near-field P-wave and the near-field R-wave within the operational period.

11. The medical instrument of claim 10, wherein the control device is further configured to perform the following steps:
within a set up period or the operational period, based on a sixth time difference value (C6) between a second sensing time (vLS_SUP) and a start time (Br_SUP) of the far-field R-wave within the set up period, wherein the second sensing time is a sensing time corresponding to a sensed event in the first in-vivo near-field cardiac electrogram acquired by the first electrode pair within a set up period, and the sensed event in the first in-vivo near-field cardiac electrogram is a near-field R-wave; and
within the operational period, estimating the start time (Br_OP) of the far-field R-wave within the operational period based on the fourth sensing time (aLS_OP), the time interval (PR_OP) between the near-field P-wave and the near-field R-wave within the operational period, and the sixth time difference value (C6).

12. The medical instrument of claim 10, wherein the control device is further configured to perform the following steps:
within a set up period, obtaining an eighth time difference value (C8) between a second sensing time (vLS_SUP) and a sensing time (GS_SUP) of a far-field R-wave within the set up period by the first electrode pair, wherein the second sensing time (vLS_SUP) is a sensing time corresponding to a sensed event in a first in-vivo near-field cardiac electrogram acquired by the first electrode pair within the set up period, and the sensed event in the first in-vivo near-field cardiac electrogram is a near-field R-wave; and
within the operational period, estimating the sensing time (GS_OP) of the far-field R-wave within the operational period based on the fourth sensing time (aLS_OP), a difference (PR_OP) between the first sensing time (vLS_OP) and the fourth sensing time (aLS_OP), and the eighth time difference value (C8).

13. The medical instrument of claim 10, wherein the control device is further configured to perform, during the operational period, the following step:
estimating the start time (Bp_OP) of a far-field P-wave within the operational period based on the fourth sensing time (aLS_OP), a start time (Bp_SUP) of a far-field P-wave within a set up period and a fifth sensing time (aLS_SUP);
wherein the fifth sensing time (aLS_SUP) is a sensing time corresponding to a sensed event in a second in-vivo near-field cardiac electrogram acquired by the second electrode pair within the set up period.

14. The medical instrument of claim 2, wherein the control device is further configured to perform the following step:
within an operational period, estimating the start time (Br_OP) or the sensing time (GS_OP) of the far-field R-wave within the operational period based on a difference value between the first sensing time (vLS_OP) and a first specific constant, wherein the first specific constant being is in the range of 0-150 ms.

15. The medical instrument of claim 1, wherein the electrode pair is a second electrode pair, the cardiac electrical sensing time is a fourth sensing time (aLS_OP), the in-vivo near-field cardiac electrogram is a second in-vivo near-field cardiac electrogram, and the sensed event is a near-field P-wave.

16. The medical instrument of claim 15, wherein the control device is further configured to perform the following steps:
within a set up period, acquiring a start time (Br_SUP) of a far-field R-wave within a set up period; and acquiring a start time (Bp_SUP) of a far-field P-wave within the set up period;
within the set up period or the operational period, acquiring a time interval (PR_SUP) between the far-field R-wave and the far-field P-wave within the set up period based on the start time (Br_SUP) of the far-field R-wave within the set up period and the start time (Bp_SUP) of the far-field P-wave within the set up period; and
within the operational period, estimating the start time (Br_OP) or the sensing time (GS_OP) of the far-field R-wave within the operational period based on the fourth sensing time (aLS_OP) and the time interval (PR_SUP) between the far-field R-wave and the far-field P-wave within the set up period.

17. The medical instrument of claim 16, wherein the control device is further configured to perform the following steps:
within the set up period or the operational period, obtaining an eleventh time difference value (C11) between a second sensing time (vLS_SUP) and a sensing time (GS_SUP) of the far-field R-wave within the set up period; wherein the second sensing time (vLS_SUP) is a sensing time corresponding to a sensed event in a first in-vivo near-field cardiac electrogram acquired by a first electrode pair within the set up period, and the sensed event in the first in-vivo near-field cardiac electrogram is a near-field R-wave; obtaining a twelfth constant (C12) based on the time interval (PR_SUP) between the far-field R-wave and the far-field P-wave within the set up period and the eleventh time difference value (C11); and
estimating the sensing time (GS_OP) of the far-field R-wave within the operational period based on the fourth sensing time (aLS_OP) and the twelfth constant (C12) within the operational period.

18. The medical instrument of claim 16, wherein the control device is further configured to perform the following steps:
within the set up period or the operational period, obtaining a thirteenth time difference value (C13) between a second sensing time (vLS_SUP) and the start time (Br_SUP) of the far-field R-wave within the set up period; wherein the second sensing time (vLS_SUP) is a sensing time corresponding to a sensed event in a first in-vivo near-field cardiac electrogram acquired by a first electrode pair within the set up period, and the sensed event in the first in-vivo near-field cardiac electrogram is a near-field R-wave; obtaining a fourteenth constant (C14) based on the time interval (PR_SUP) between the far-field R-wave and the far-field P-wave within the set up period and the thirteenth time difference value (C13); and
within the operational period, estimating the start time (Br_OP) of the far-field R-wave within the operational period based on the fourth sensing time (aLS_OP) and the fourteenth constant (C14).

19. The medical instrument of claim 15, wherein the control device is further configured to perform the following step:
within an operational period, estimating the start time (Br_OP) or the sensing time (GS_OP) of the far-field R-wave within the operational period based on a difference value between the fourth sensing time (aLS_OP) and a second specific constant, wherein the second specific constant is in the range of 0-350 ms.

20. The medical instrument of any one of claims 3-9, 11-13 and 16-18, wherein a heartbeat generated during the operational period and a heartbeat generated during the set up period result from the same atrial or ventricular electrical activity;
wherein the atrial or ventricular electrical activity is any one of: a sinus heartbeat, a ventricular heartbeat resulting from atrial conduction, a ventricular heartbeat resulting from ventricular pacing, and a ventricular heartbeat resulting from atrial pacing.

21. The medical instrument of any one of claims 1-20, wherein the control device is further configured to perform, during the operational period, the following steps:
estimating a sensing time window corresponding to a far-field R-wave based on the estimated start time (Br_OP) or sensing time (GS_OP) of the far-field R-wave within the operational period; and
determining whether to deliver a CCM pulse based on the sensing time window and the cardiac electrical sensing time.

22. The medical instrument of any one of claims 1-20, wherein the control device is further configured to perform, during the operational period, the following steps:
estimating a pulse delivery time window corresponding to a far-field R-wave based on the estimated start time (Br_OP) or sensing time (GS_OP) of the far-field R-wave within the operational period; and
determining whether to deliver a CCM pulse based on the pulse delivery time window and the cardiac electrical sensing time.

23. The medical instrument of claim 1, further comprising at least one ventricular electrode lead configured with the electrode pair for sensing, the electrode pair being a first electrode pair; or further comprising at least one atrial electrode lead configured with the electrode pair for sensing, the electrode pair being a second electrode pair.

24. The medical instrument of claim 1, wherein the electrode pair is a first electrode pair or a second electrode pair is used for attachment to a ventricle or an epicardium of the ventricle.
